# EUROPEAN PATENT SPECIFICATION

(11) **EP 3 392 291 B1**
(45) Date of publication and mention of the grant of the patent: **05.08.2020**
(21) Application number: 16876045.2
(22) Date of filing: 15.12.2016
(51) Int. Cl.: C08G 69/36, C08G 69/48, A61K 35/60

(54) **VACCINE ADJUVANT COMPOSITION BASED ON AMPHIPHILIC POLYAMINO ACID POLYMER, CONTAINING SQUALENE**
IMPFSTOFFADJUVANSZUSAMMENSETZUNG AUF BASIS VON AMPHIPHILEM POLYAMINOSÄUREPOLYMER MIT SQUALEN
COMPOSITION D'ADJUVANT POUR VACCIN À BASE DE POLYMÈRE AMPHIPHILE DE POLYACIDE AMINÉ, CONTENANT DU SQUALÈNE

(30) Priority: 16.12.2015 KR 20150180413
(43) Date of publication of application: 24.10.2018
(73) Proprietor: Huvet Bio, Inc., Seoul 06247 (KR)
(72) Inventor: HAAM, Seungjoo, Seoul 06670 (KR); LIM, Jong Woo, Seoul 03722 (KR); KIM, Hyun-Ouk, Seoul 03722 (KR); CHOI, Jihye, Incheon 22165 (KR); YUN, Da Yeon, Seoul 03722 (KR); KIM, Jihye, Seoul 03722 (KR); SONG, Daesub, Daejeon 34069 (KR); YEOM, Min Joo, Sejong 30019 (KR); NA, Woon Sung, Sejong 30019 (KR)
(74) Representative: Schiweck Weinzierl Koch Patentanwälte Partnerschaft mbB
(86) International application number: PCT/KR2016/014714
(87) International publication number: WO 2017/105103

(56) References cited:
- CN-A- 101 433 718
- KR-A- 20080 091 808
- US-A1- 2002 128 177
- US-A1- 2008 206 183
- US-A1- 2012 088 848
- TIMOTHY J. DEMING: "Synthesis of Side-Chain Modified Polypeptides", CHEMICAL REVIEWS, vol. 116, no. 3, 6 July 2015 (2015-07-06), pages 786-808, XP055591880, US ISSN: 0009-2665, DOI: 10.1021/acs.chemrev.5b00292
- RODRIGUEZ-HERNANDEZ, J. ET AL.: 'Highly Branched Poly (L-lysine)' BIOMACROMOLECULES vol. 4, no. 2, 2003, pages 249 - 258, XP002250742
- SUN, J. ET AL.: 'Direct Formation of Giant Vesicles from Synthetic Polypeptides' LANGMUIR vol. 23, no. 16, 2007, pages 8308 - 8315, XP055397701
- GJETTING, T. ET AL.: 'Effective Nanoparticle-based Gene Delivery by a Protease Triggered Charge Switch' ADVANCED HEALTHCARE MATERIALS vol. 3, no. 7, 2014, pages 1107 - 1118, XP055397705
- SEUBERT, A. ET AL.: 'Adjuvanticity of the Oil-in-water Emulsion MF59 is Independent of Nlrp3 Inflammasome but Requires the Adaptor Protein MyD88' PROCEEDINGS OF THE NATIONAL ACADEMY OF SCIENCES THE UNITED STATES OF AMERICA vol. 108, no. 27, 2011, pages 11169 - 11174, XP055397706

## Description

### [Technical Field]

The present invention relates to an amphiphilic poly-amino acid polymer, an immunoadjuvant composition including emulsion particles containing the polymer, a vaccine composition including an antigen and the polymer emulsion particles, and a method of preparing the same.

### [Background Art]

Adjuvants can be used for the development of a vaccine through an increase in the antigenicity thereof or for disease treatment and prevention through an increase in a nonspecific immune response thereof to an antigen. Since immunoadjuvants can rapidly and strongly maintain an immune response to an antigen for a long time when the amount of antigen is small, they are used in vaccine production. In addition, by using specific immunoadjuvants or varying the amounts of antigens, an immune response, the type of antigen-specific antibody or a subtype thereof, etc. can be controlled. By using immunoadjuvants, the efficacy of a vaccine can be increased and protection against a variety of viral infections can be accomplished. The development of such immunoadjuvants is accelerating with the development of vaccines, and, as the scope of immune-related diseases is widening, development of new immunoadjuvants is considered promising.

However, surfactants used in existing immunoadjuvants have problems such as relatively low biocompatibility and poor stability. Therefore, to address such problems, the development of a more biocompatible and stable immunoadjuvant is required.

### [Disclosure]

### [Technical Problem]

Therefore, the present invention has been made in view of the above problems, and it is one object of the present invention to provide an immunoadjuvant composition and a vaccine composition that are biocompatible and exhibit a high antibody titer, and methods of preparing the same.

### [Technical Solution]

In accordance with an aspect of the present invention, the above and other objects can be accomplished by the provision of an amphiphilic poly-amino acid polymer.

In accordance with another aspect of the present invention, there is provided a method of preparing the amphiphilic poly-amino acid polymer.

In accordance with still another aspect of the present invention, there is provided an immunoadjuvant composition including squalene-carrying amphiphilic polymer particles in an oil-in-water emulsion state that contain the amphiphilic poly-amino acid polymer.

In accordance with yet another aspect of the present invention, there is provided a method of preparing an immunoadjuvant composition, the method including a step of mixing an aqueous solution including the amphiphilic poly-amino acid polymer with a solution including squalene.

In accordance with yet another aspect of the present invention, there is provided vaccine composition including an antigen; and squalene-carrying amphiphilic polymer particles in an oil-in-water emulsion state that contain the amphiphilic poly-amino acid polymer.

In accordance with yet another aspect of the present invention, there is provided a method of preparing a vaccine composition, the method including a step of mixing a solution including an antigen with an emulsion solution including the squalene-carrying amphiphilic poly-amino acid polymer.

Hereinafter, the configuration of the present invention will be particularly described.

The present invention provides an amphiphilic poly-amino acid polymer represented by Formula 1 below: 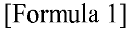 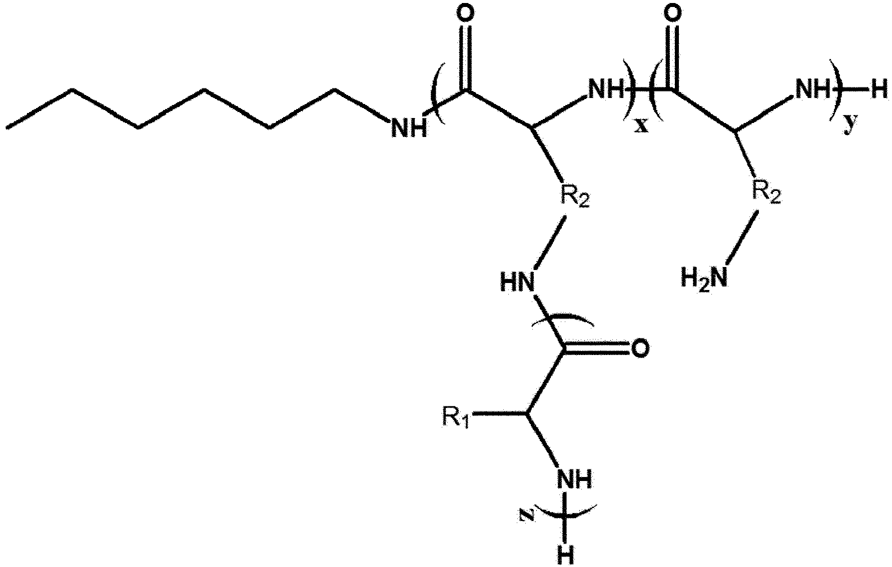 wherein R₁ is one selected from the group consisting of 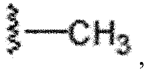 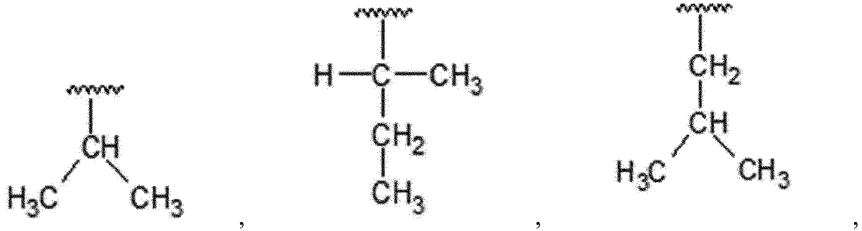 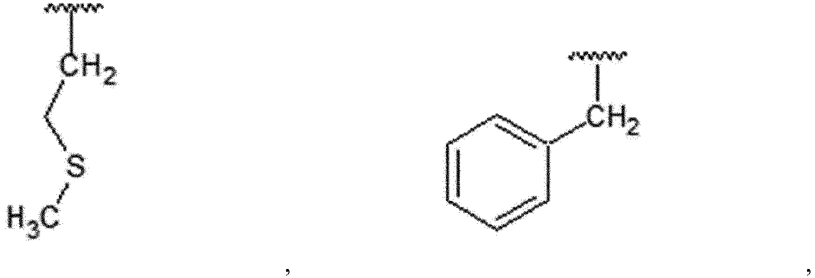 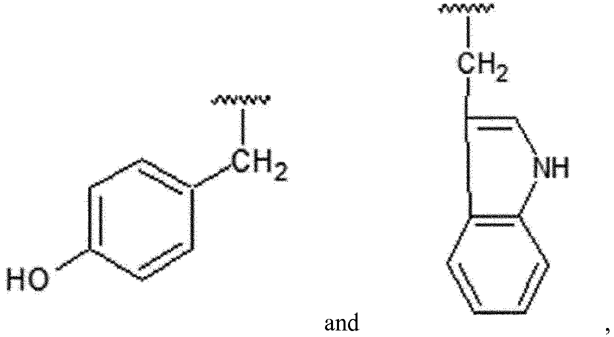 R₂ is 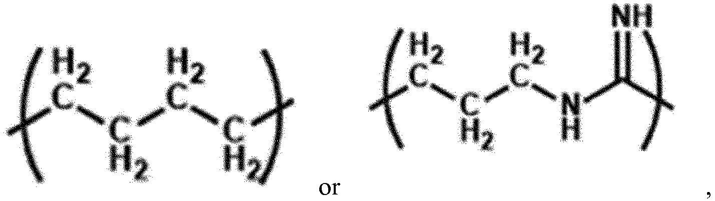 and
x, y, and z are integers greater than 1.

In an embodiment, the amphiphilic poly-amino acid polymer may include an amphiphilic poly-amino acid polymer wherein the R₁ is 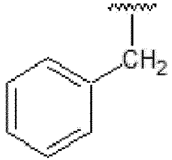 and the R₂ is

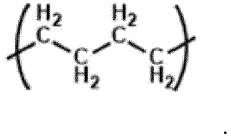

In addition, the present invention provides a method of preparing an amphiphilic poly-amino acid polymer, the method including a step of mixing a compound represented by Formula 2 below with a compound represented by Formula 3 below to prepare an amphiphilic poly-amino acid polymer represented by Formula 1 below: 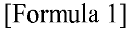 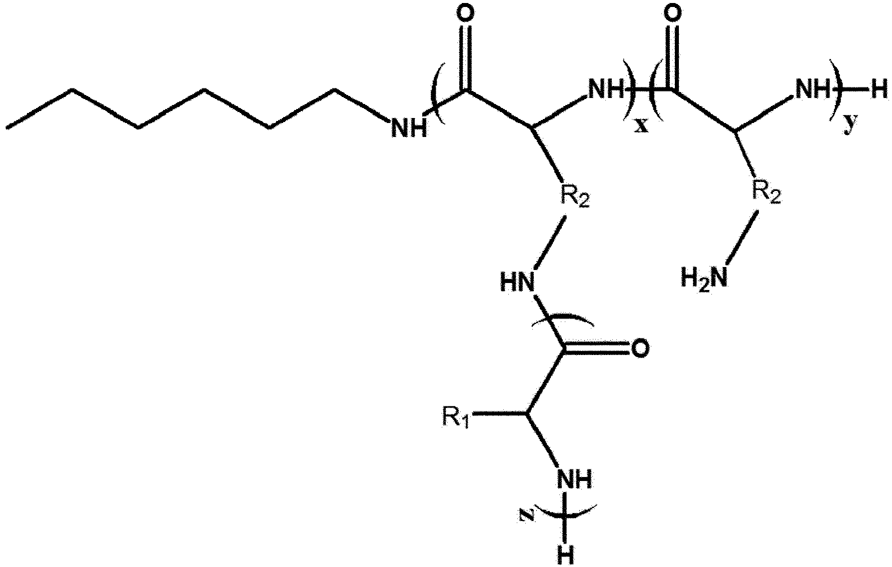 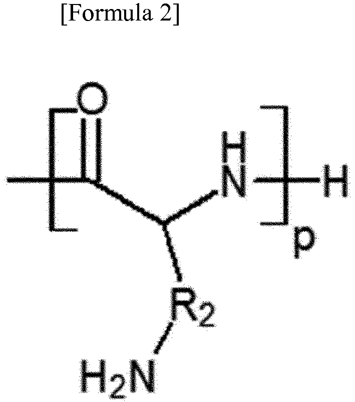 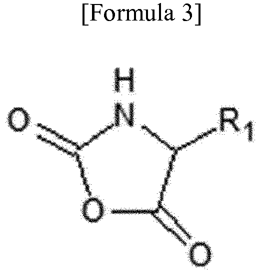 wherein, in Formulas 1 to 3, R₁ is one selected from the group consisting of 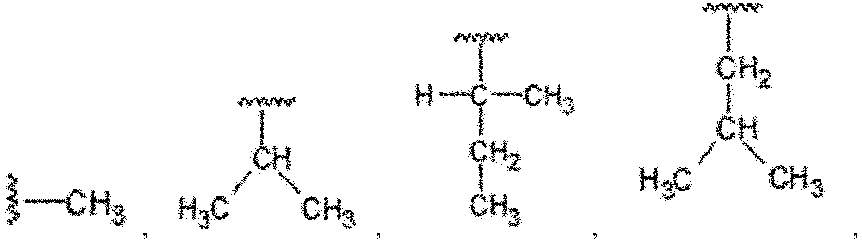 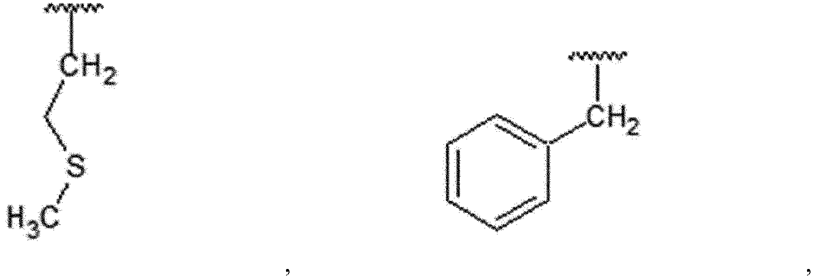 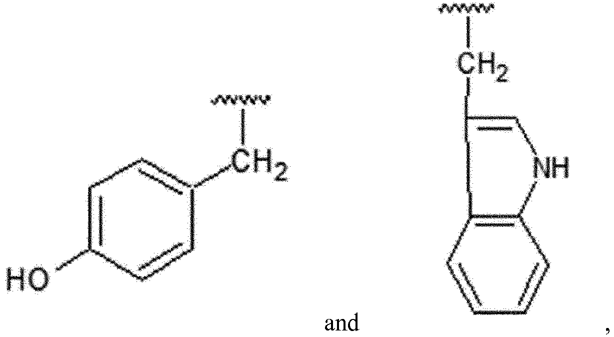 R₂ is 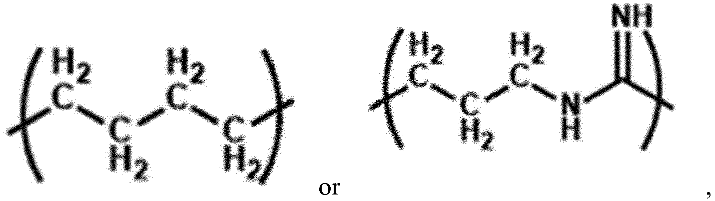 and
x, y, z, and p are integers greater than 1.

In the present invention, x and y in Formula 1 may be included in a ratio of 1:0.1 to 1:10, for example 1:0.5 to 1:5 or 1:0.5 to 1:2, preferably 1:1. When a molar ratio of the compound represented by Formula 2 to the compound represented by Formula 3 is adjusted within these ranges, the amphiphilicity of the amphiphilic poly-amino acid may be maintained.

In an embodiment, the amphiphilic poly-amino acid polymer may be present in a micelle form characterized by a spherical particle shape composed of a hydrophobic core and a hydrophilic shell or in a polymersome form composed of a hollow hydrophilic core and hydrophobic and hydrophilic shells doubly enclosing the hollow hydrophilic core, due to the amphiphilic characteristics thereof.

According to an embodiment of the present invention, the compound represented by Formula 2 and the compound represented by Formula 3 are dissolved in an organic solvent such that a ratio of x to y in Formula 1 is 1:0.1 to 1:10, followed by performing polymerization at 30°C to 40°C for 24 hours to 60 hours, e.g., for 48 hours. A polymer is isolated from the resultant mixture by a precipitation method, followed by being sufficiently dialyzed and then being lyophilized. As a result, an amphiphilic poly-amino acid polymer having a branched structure may be obtained.

The present invention also provides an immunoadjuvant composition including the squalene-carrying amphiphilic polymer particles in an oil-in-water emulsion state that contain the amphiphilic poly-amino acid polymer.

With regard to the immunoadjuvant composition, all of the aforementioned contents of the amphiphilic poly-amino acid polymer may be applied.

In the present invention, the amphiphilic polymer particles in an oil-in-water emulsion state may be a squalene-carrying amphiphilic poly-amino acid polymer. Here, squalene functions as an adjuvant or immunoadjuvant and is used to amplify the effect of a vaccine.

In the present invention, the term "immunoadjuvant" refers to a substance called an immunoadjuvant or adjuvant. More particularly, the immunoadjuvant refers to a substance that helps a large amount of an antibody to be generated through an immune system by increasing the response to a vaccine. The squalene is generally extracted from the liver of deep sea sharks, but may be produced through other various routes without being specifically limited.

In an embodiment, the content of squalene, which is carried in the amphiphilic poly-amino acid polymer, may be 0.5% (v/v) to 10% (v/v), for example, 1% (v/v) to 8% (v/v), 3% (v/v) to 7% (v/v), or 4% (v/v) to 6% (v/v), preferably 5% (v/v). When the content of squalene is adjusted within these ranges, the squalene may function as an immunoadjuvant.

In an embodiment, a molar ratio of the squalene to the amphiphilic poly-amino acid polymer may be 1:0.001 to 1:0.999, for example 1:0.001 to 0.5, or 1:0.001 to 1:0.005.

When a molar ratio of the squalene to the amphiphilic poly-amino acid polymer is adjusted within these ranges, the sizes of the amphiphilic polymer particles in an oil-in-water emulsion state may be controlled.

The amphiphilic poly-amino acid polymer according to the present invention has high biocompatibility due to the application of an amino acid polymer, thereby being suitable as an immunoadjuvant.

In addition, the amphiphilic polymer particles in an oil-in-water emulsion state may have an average diameter of 10 nm to 1 *µ*m, for example 100 nm to 500 nm or 200 nm to 400 nm.

Since the immunoadjuvant composition of the present invention includes the amphiphilic poly-amino acid polymer instead of a surfactant used in conventional immunoadjuvant compositions, it is more biocompatible and has a high antibody titer.

With regard to the method, all of the aforementioned contents may be applied to the amphiphilic poly-amino acid polymer, the amphiphilic polymer particles in an oil-in-water emulsion state, and the immunoadjuvant.

The present invention also provides a method of preparing an immunoadjuvant composition, the method including a step of mixing an aqueous solution including the amphiphilic poly-amino acid polymer with a solution including squalene to prepare amphiphilic polymer particles in an oil-in-water emulsion state.

With regard to the method, all of the aforementioned contents may be applied to the amphiphilic poly-amino acid polymer, the amphiphilic polymer particles in an oil-in-water emulsion state, the immunoadjuvant, and the squalene.

The present invention also provides a vaccine composition including an antigen; and the squalene-carrying amphiphilic polymer particles in an oil-in-water emulsion state that contain the amphiphilic poly-amino acid polymer.

In an embodiment, the antigen may be an influenza virus antigen or a porcine epidemic diarrhea virus antigen, but the present invention is not limited thereto. The viral antigens are not specifically limited as long as they exhibit immune enhancement effects when squalene, as an immunoadjuvant, is administered along with a vaccine.

The vaccine composition of the present invention may include a pharmaceutically acceptable carrier. In the present invention, "pharmaceutically acceptable carrier" refers to a carrier or diluent that does not significantly stimulate the organism and does not interfere with the biological activity and properties of an administered ingredient. In the present invention, the pharmaceutically acceptable carrier may be saline, sterilized water, Ringer's solution, buffered saline, a dextrose solution, a maltodextrin solution, glycerol, ethanol or a mixture of one or more thereof, and may be formulated in the form of an injection suitable for injection into the tissues and the organs through addition of general additives, such as an antioxidant, a buffer, and a bacteriostatic agent, as needed. In addition, the vaccine composition may be formulated into a dry preparation (particularly, a lyophilized formulation), which is capable of being prepared into an injectable solution, by adding an isotonic sterile solution or, as needed, sterile water or physiological saline. In addition, the carrier may be bound to a target organ-specific antibody specifically acting on the target organ or other ligands.

Preferably, the composition of the present invention may further include a filler, an excipient, a disintegrant, a binder, a lubricant, and the like. In addition, the composition of the present invention may be formulated using a method known in the art to provide rapid, sustained, or delayed release of active ingredients after administration to a mammal.

In the present invention, the term "administration" refers to administration of the composition of the present invention to a patient by any appropriate method. The composition of the present invention may be administered through various routes, such as oral or parenteral administration, so long as it can reach a target tissue. The composition of the present invention may be applied through intraperitoneal, intravenous, intramuscular, subcutaneous, intradermal, oral, topical, intranasal, intrapulmonary, or rectal administration, but the present invention is not limited thereto.

In the specification, the term "effective amount" refers to an amount necessary to delay or totally stop the onset or progression of a specific disease to be treated. In the present invention, the composition may be administered in a pharmaceutically effective amount. It will be apparent to those skilled in the art that an appropriate total daily dose may be determined by a practitioner within the scope of sound medical judgment.

For the purposes of the present invention, a therapeutically effective amount for a specific patient preferably depends various factors including the type and degree of response to be achieved, particular compositions including other formulations or not, the age, weight, physical condition, sex, and diet of a patient, administration time, administration route, and a secretion rate of a composition, treatment duration, drugs used or co-used with a specific composition, and similar factors well known in the art of medicine.

The present invention also provides a method of preparing a vaccine composition, the method including a step of mixing a solution including an antigen and an emulsion solution including the squalene-carrying amphiphilic poly-amino acid polymer.

The vaccine composition may be prepared by mixing the solution including an antigen and the emulsion solution in a volumetric ratio of 1:0.2 to 1:5, e.g., in a volumetric ratio of 1:1.

### [Advantageous Effects]

As described above, an immunoadjuvant composition of the present invention is prepared using an amphiphilic poly-amino acid polymer, thereby providing a vaccine immunoadjuvant composition which is more biocompatible and has a high antibody titer.

### [Description of Drawings]

FIG. 1 illustrates a synthesis process of an amphiphilic poly-amino acid polymer (PLL-b-Phe).
FIG. 2 illustrates a TEM image of an oil-in-water emulsion.
FIG. 3 illustrates a diagrammatic view of an oil-in-water emulsion.
FIG. 4 is a diagrammatic view illustrating a preparation method of an oil-in-water emulsion.
FIG. 5 illustrates a result for confirming a polymerization result of polylysine (PLL) through NMR analysis.
FIG. 6 illustrates a result for confirming a polymerization result of an amphiphilic poly-amino acid polymer (PLL-b-Phe) through NMR analysis.
FIG. 7 is a diagrammatic view illustrating a polymerization process of an amphiphilic poly-amino acid polymer (PLL-b-Phe).
FIG. 8 illustrates a results of emulsion size change dependent upon the amount of amphiphilic poly-amino acid polymer (PLL-b-Phe)-carried squalene (1 % / 200 µl) investigated by a DLS method.
FIG. 9 illustrates a results of an emulsion size change dependent upon the amount of amphiphilic poly-amino acid polymer (PLL-b-Phe)-carried squalene (5 % / 1 ml) investigated by a DLS method.
FIG. 10 illustrates results of emulsion size change dependent upon the amount of an amphiphilic poly-amino acid polymer (PLL-b-Phe)-carried squalene (10 % / 2 ml) investigated by a DLS method.
FIG. 11 illustrates HI titer values measured through mouse erythrocyte aggregation inhibition experiments conducted to measure an antibody titer of a vaccine composition of the present invention under the following five conditions: (i) PBS (non-vac), ii) an influenza vaccine antigen (Ag-only), iii) a combination of an influenza vaccine antigen and a commercially available immunoadjuvant for research (AddaVax), iv) a combination of an influenza vaccine antigen and PLL-b-Phe (Amino-Sq), and v) a combination of an influenza vaccine antigen and an addavax produced in a laboratory (T80-Sq)).
FIG. 12 illustrates a set of results for confirming the stability of particles dependent upon a storage temperature and period of amphiphilic amino acid polymer particles in an oil-in-water emulsion state.
FIG. 13 illustrates an ELISA result of IgG of serum collected from each of mouse models to measure the immunological activity of a vaccine composition of the present invention.
FIG. 14 illustrates an ELISA result of IgG1 of serum collected from each of mouse models to measure the immunological activity of a vaccine composition of the present invention.
FIG. 15 illustrates an ELISA result of IgG2a of serum collected from each of mouse models to measure the immunological activity of a vaccine composition of the present invention.
FIG. 16 illustrates weight measurement results of mouse models challenge-inoculated with an influenza virus (H1N1) to evaluate an infection inhibition ability of a vaccine composition according to the present invention.
FIG. 17 illustrates an antibody titer measurement result of virus collected from the lungs of each of mouse models at the 5th and 7th days of influenza virus infection.
FIG. 18 illustrates an ELISA result of IgG of serum collected from each of guinea pig models to measure the immunological activity of a vaccine composition according to the present invention.

### [Best Mode]

Hereinafter, the present application will be described in more detail with reference to the following examples. These examples are provided for illustrative purposes only and should not be construed as limiting the scope and spirit of the present application.

### [Example 1] Preparation of emulsion including squalene-carrying amphiphilic poly-amino acid polymer (PLL-b-Phe)

### 1) Synthesis of amphiphilic poly-amino acid polymer (PLL-b-Phe)

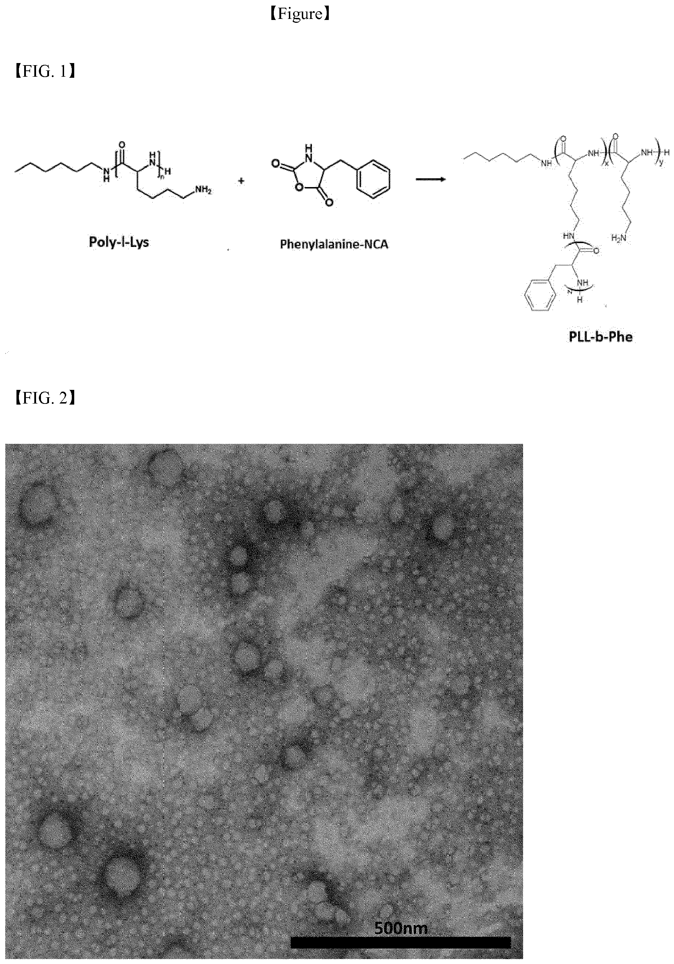

To polymerize a branched amphiphilic poly-amino acid polymer (PLL-b-Phe) of phenylalanine-NCA (Phe-NCA) which is an anhydrous hydrophobic amino acid with polylysine (Poly-Lys) which is a cationic poly-amino acid polymer, a polymerization degree (n) of Poly-Lys was found, and the masses thereof were determined such that a molar ratio of an amine group (-NH₂) of Poly-Lys to phenylalanine-NCA (Phe-NCA) became 1:0.9 to 0.1. The samples were dissolved in 25 mL of dimethylformamide (DMF) to prepare a solution mixture, followed by performing polymerization at 35°C for 48 hours. A polymer was isolated from the reaction-terminated solution mixture using ethyl ether by a precipitation method, and was sufficiently dialyzed to remove the excess solvent and samples therefrom, and then lyophilized. As a result, an amphiphilic poly-amino acid polymer (PLL-b-Phe), in which Poly-Lys and Phe-NCA were polymerized in a branched form, was obtained. A synthesis process of PLL-b-Phe is illustrated in FIG. 1.

### 2) Preparation of oil-in-water emulsion

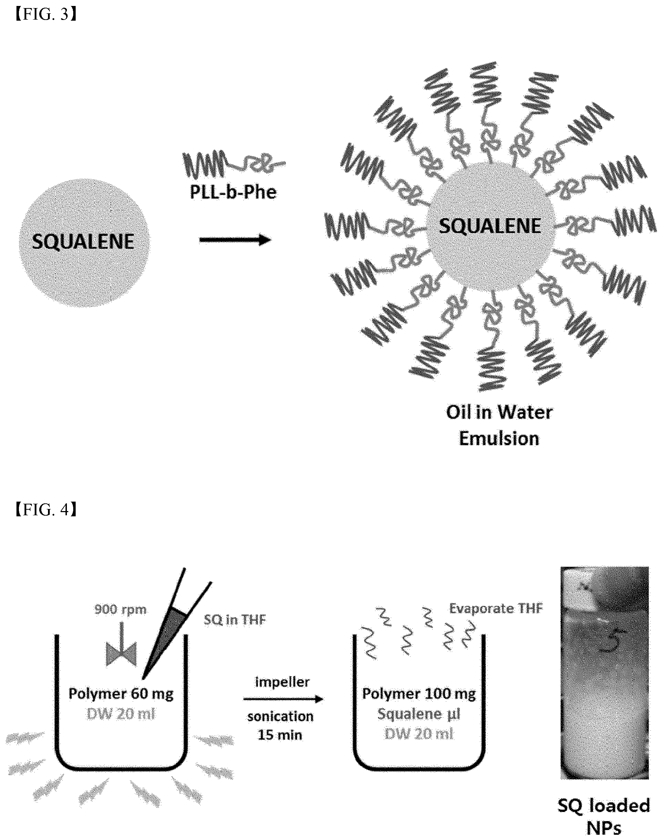

40 mg of the amphiphilic poly-amino acid polymer (PLL-b-Phe) obtained in step 1) was dissolved in 20 mL of distilled water (DW), thereby preparing an aqueous solution. The prepared aqueous solution was sonicated, and stirred at 800 rpm. A squalene solution mixture, in which squalene was dissolved in a small amount of tetrahydrofuran (THF), was added batchwise to the aqueous solution while stirring the aqueous solution. The solution mixture containing squalene was sonicated for 30 minutes, and stirred at 300 rpm to 400 rpm for 5 hours or more to remove the THF solvent therefrom. Subsequently, a PLL-b-Phe emulsion in an oil-in-water state, in which squalene was carried, was prepared. FIG. 2 illustrates a photograph of the prepared emulsion observed with a TEM microscope. FIGS. 3 and 4 illustrate diagrammatic views of the emulsion in an oil-in-water state and the method of preparing the emulsion.

### 3) Preparation of vaccine composition

25 µl of the emulsion in an oil-in-water state prepared in step 2) was mixed with 25 µl of a PBS solution including an influenza virus antigen in a volumetric ratio of about 1:1 such that an HA titer became about 128, thereby preparing a vaccine composition.

### [Experimental Example 1] NMR analysis

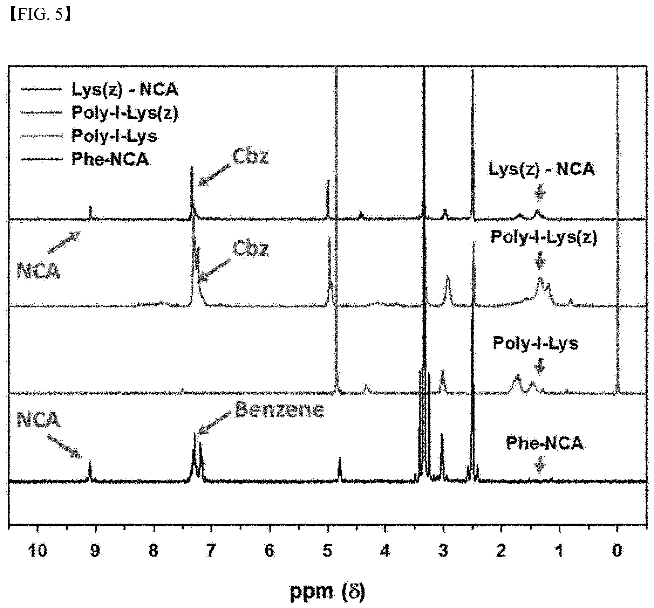

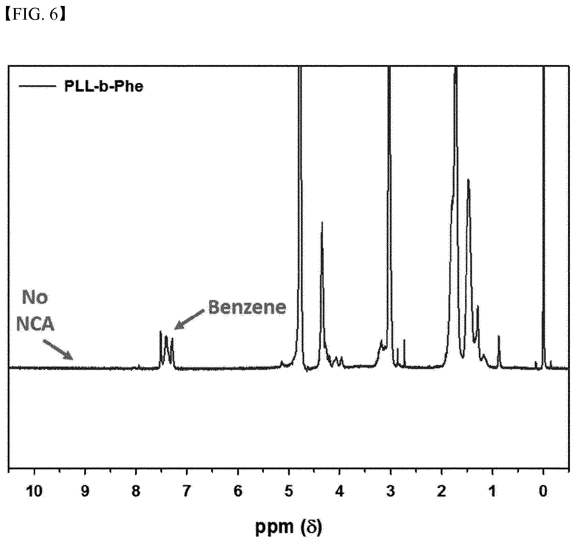

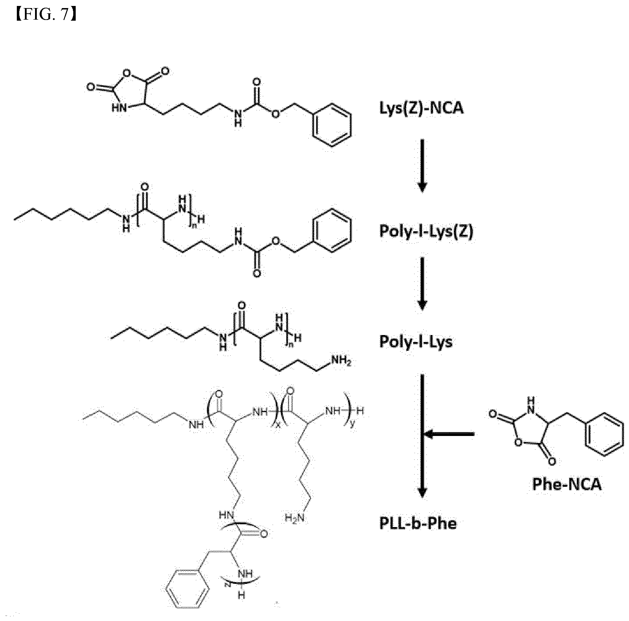

A polymerization result of the amphiphilic poly-amino acid polymer (PLL-b-Phe) was investigated through NMR analysis. Results are shown in FIGS. 5 and 6. FIG. 7 illustrates a diagrammatic view of a polymerization process of PLL-b-Phe. First, H-Lys(Z)-OH was reacted with triphosgene to synthesize a Lys(z)-NCA lysine anhydride. NCA and Cbz peaks of the synthesized Lys(z)-NCA lysine anhydride were observed through NMR analysis to confirm whether it was suitably synthesized. The synthesized Lys(z)-NCA lysine anhydride was polymerized using hexylamine to synthesize a cationic poly-amino acid polymer (PLL(z), Poly-1-lysine(z)). Successful polymerization of the PLL(z) was confirmed through the disappearance of NCA peaks in NMR data. Subsequently, a Cbz protective group protecting an amine group was de-protected using an HBr/acetic acid solution, and synthesis of polylysine (Poly-1-lysine), as a cationic amino acid polymer, was confirmed through weakening and disappearance of Cbz peaks in NMR analysis data. In addition, a hydrophobic amino acid anhydride (Phe-NCA) was reacted with polylysine so that a phenylalanine anhydride was polymerized in a branch shape to the polylysine. As a result, polymerization was confirmed as being accomplished through NMR analysis data wherein the NCA peaks disappeared and a benzene ring peak of phenylalanine was generated.

### [Experimental Example 2] Measurement of size distribution of amphiphilic polymer particles in oil-in-water-type emulsion state by DLS method

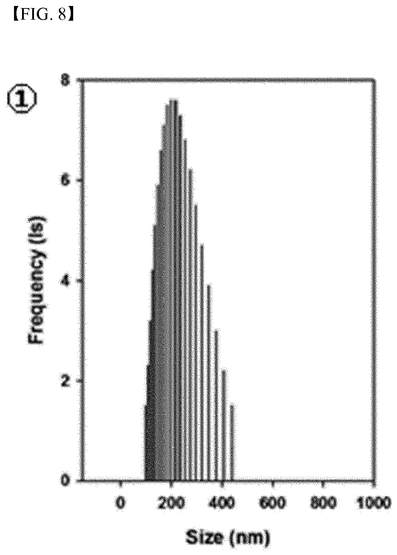

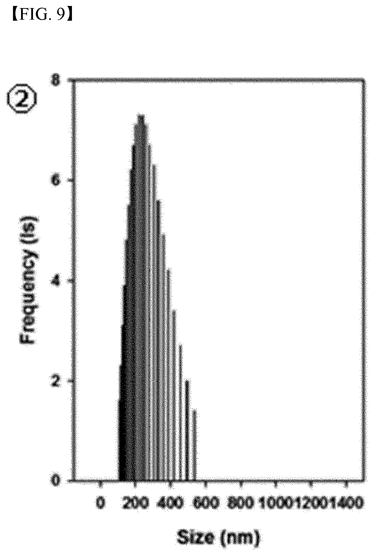

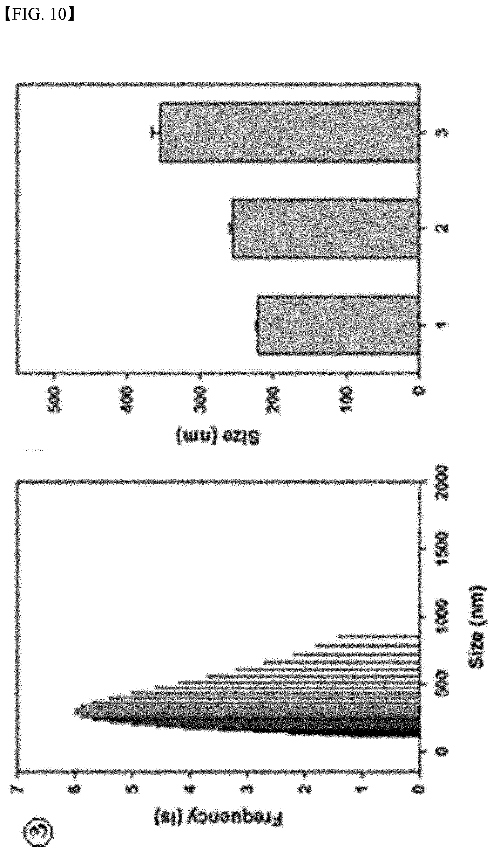

The amount of amphiphilic poly-amino acid polymer (PLL-b-Phe) was fixed and the amount of squalene was adjusted to measure a size distribution of the amphiphilic polymer particles in an oil-in-water-type emulsion state. Using the emulsion preparation method of step 2) of Example 1, squalene was carried in the polymer. Particle size changes dependent upon the amount of squalene were investigated using a dynamic light scattering (DLS) method. Results are shown in FIGS. 8 to 10 and Table 1 below.

**[Table 1]**

| | D.W (mL) | PLL-b-Phe (mg) | Squalene | RPM | Size (nm, AVG) | STDEV |
|---|---|---|---|---|---|---|
| ① | 20 | 60 | 1% / 200 µl | 900 | 221.03 | 1.87 |
| ② | 20 | 60 | 5% / 1 mL | 900 | 255.11 | 4.25 |
| ③ | 20 | 60 | 10% / 2 mL | 900 | 354.47 | 11.77 |

As shown in FIGS. 8 to 10 and the Table 1, it was confirmed that the sizes of the amphiphilic polymer particles in an oil-in-water emulsion state were gradually increased with increasing squalene content. In addition, it was confirmed that the particles were evenly monodispersed through the DLS analysis.

### [Experimental Example 3] Measurement of antibody titer

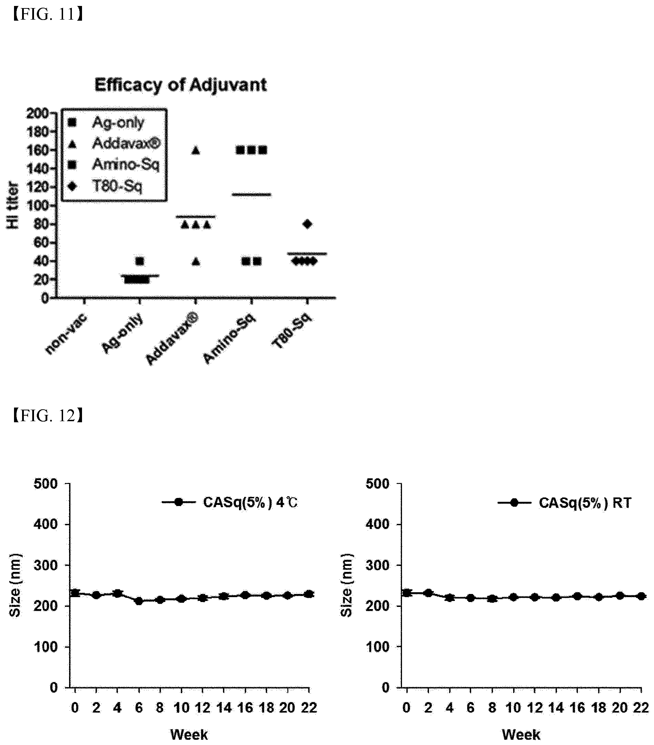

Mice were intramuscularly inoculated at 2-week intervals and, four weeks after the first inoculation, were subjected to an erythrocyte coagulation inhibition experiment to investigate antibody titers. The antibody titers were measured using a hemaglutination inhibition (HI) test method. Experiments were carried out under five conditions: (i) PBS (non-vac of FIG. 11), ii) an influenza vaccine antigen (Ag-only of FIG. 11), iii) a combination of an influenza vaccine antigen and a commercially available immunoadjuvant, AddaVax, for research (AddaVax of FIG. 11), iv) a combination of an influenza vaccine antigen and PLL-b-Phe (Amino-Sq of FIG. 11), v) a combination of an influenza vaccine antigen and an addavax produced in a laboratory (T80-Sq of FIG. 11). In each of the conditions, five mice were used as subjects, and a CA04 (H1N1) virus was used as an immune vaccine antigen. Results are shown in FIG. 11.

As shown in FIG. 11, it was confirmed that, when an antibody titer was confirmed as an average HI titer value, an antibody titer of the combination of the influenza vaccine antigen and PLL-b-Phe was 1.5 times higher than that of the combination of the influenza vaccine antigen and addavax.

### [Experimental Example 4] Confirmation of stability of particles

A storage temperature-dependent particle size of the emulsion including the amphiphilic poly-amino acid polymer (PLL-b-Phe), in which squalene was carried, prepared in step 2) of Example 1 was measured by means of a DLS instrument to investigate the stability of the particles. To investigate storage period-dependent stability of the particles, the particles were stored in a 4°C refrigerator and at room temperature (RT, 25°C).

As shown in FIG. 12, it was confirmed that the initial particle size was maintained even if a storage period was prolonged. From this result, it can be confirmed that the amphiphilic amino acid polymer particles according to the present invention are uniformly dispersed even when stored for a long time.

### [Experimental Example 5] Measurement of immunological activity in virus-infected models

### 1) Measurement of immunological activity in influenza virus (H1N1)-infected mouse models

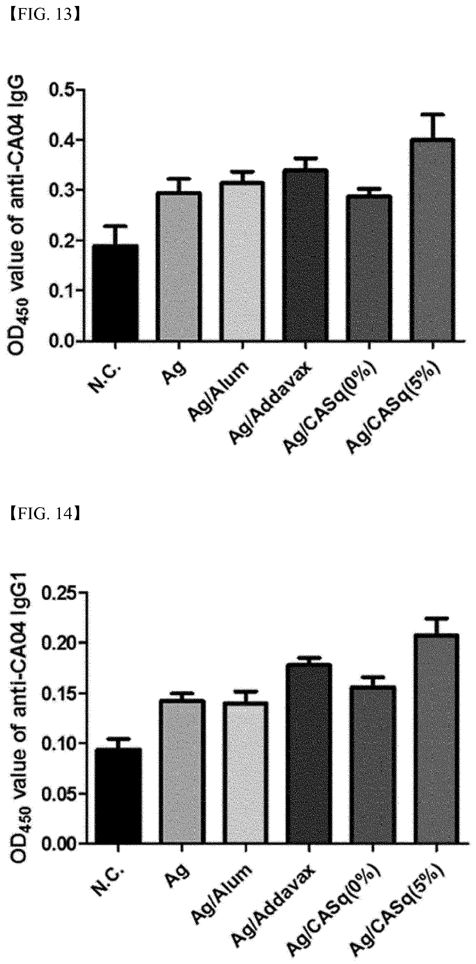

Mice were intramuscularly inoculated twice at 2-week intervals. Two weeks after the last inoculation, blood samples were collected and subjected to immunological activity measurement experiments using ELISA to investigate an antigen-specific antibody-based immune response (humoral immunity). To measure immunological activity of the mice, each of the collected serum samples was diluted 1:50, and the amounts of IgG and isotypes thereof (IgG1, IgG2a) therein were investigated using enzyme-linked immunosorbent assay (ELISA). Results are shown in FIGS. 13 to 15. Experiments were conducted under six conditions: (i) an untreated group (N.C.: administration of PBS 50 µl), ii) an influenza vaccine antigen (Ag: administration of antigen (inactivated immune antigen, antigen titer: 32) 25 µl + PBS 25 µl), iii) a combination of an influenza vaccine antigen and a commercially available immunoadjuvant, Alum (ThermoFisher Scientific, Imject™, US) (Ag/Alum: administration of antigen (inactivated immune antigen, antigen titer: 32) 25 µl + Alum 25 µl), iv) a combination of an influenza vaccine antigen and a commercially available immunoadjuvant, AddaVax (InvivoGen, US) (Ag/AddaVax: administration of antigen (inactivated immune antigen, antigen titer: 32) 25 µl + AddaVax 25 µl), v) a combination of an influenza vaccine antigen and squalene (0%)-carrying PLL-b-Phe (Ag/CASq (0%): administration of antigen (inactivated immune antigen, antigen titer: 32) 25 µl + CASq (0%) 25 µl), and vi) a combination of an influenza vaccine antigen and squalene (5%)-carrying PLL-b-Phe (Ag/CASq (5%): administration of antigen (inactivated immune antigen, antigen titer: 32) 25 µl + CASq (5%) 25 µl). Here, the antigen and the immunoadjuvant were mixed in a ratio of 1:1 to prepare a vaccine formulation. For each of the conditions, three mice were subjected to the experiment, and a CA04 (influenza virus H1N1) virus was used as an immune vaccine antigen. Results are shown in FIGS. 13 to 15.

As shown in FIGS. 13 to 15, it was confirmed that, as results of ELISA experiments conducted to indirectly investigate the amount of IgG at absorbance (450 nm), CASq (5%) showed a higher amount of IgG than AddaVax and Alum through a difference in absorbance intensity. In addition, it was confirmed that, from results for IgG2a related to the Th1 immune response and IgG1 related to the Th2 immune response, CASq (5%) exhibited significantly higher levels.

### 2) Measurement of infection inhibition in influenza virus (H1N1)-infected mouse models

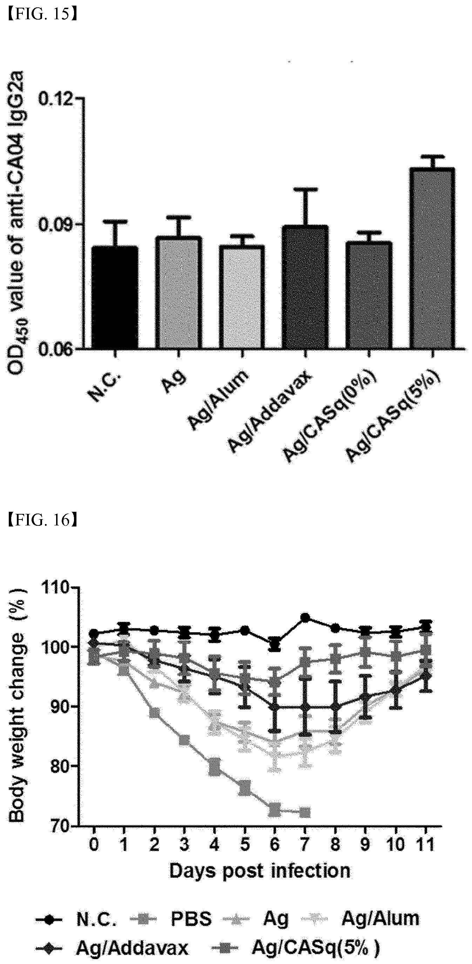

Mice were intramuscularly inoculated at 2-week intervals, and then an influenza virus (H1N1) was nasally challenge-inoculated thereto in an content of 20^{∗}LD₅₀ at a dose of 30 mℓ to investigate an infection inhibition ability of the vaccine according to the present invention. Results are shown in FIG. 16. To investigate the infection inhibition ability, the weights of the animals were measured for 11 days after the virus infection. Here, 10 mice were employed for each of the following six conditions: (i) an untreated group (N.C. of FIG. 16), ii) a PBS-treated group (PBS of FIG. 16: challenge-inoculation after administration of PBS 50 µl), iii) an influenza vaccine antigen (Ag of FIG. 16: challenge-inoculation after administration of antigen (inactivated immune antigen, antigen titer: 32) 25 µl + PBS 25 µl), iv) a combination of an influenza vaccine antigen and a commercially available immunoadjuvant Alum (ThermoFisher Scientific, Imject™, US) (Ag/Alum of FIG. 16: challenge-inoculation after administration of an antigen (inactivated immune antigen, antigen titer 32) 25 µl + Alum 25 µl), v) a combination of an influenza vaccine antigen and a commercially available immunoadjuvant AddaVax (InvivoGen, US) (Ag/AddaVax of FIG. 1: challenge-inoculation after administration of an antigen (inactivated immune antigen, antigen titer 32) 25 µl + AddaVax 25 µl), and vi) a combination of an influenza vaccine antigen and squalene (5%)-carrying PLL-b-Phe (Ag/CASq (5%) of FIG. 16: challenge-inoculation after administration of an antigen (inactivated immune antigen, antigen titer: 32) 25 µl + CASq (5%) 25 µl). Here, the antigen and the immunoadjuvant were mixed in a ratio of 1:1 to prepare vaccine formulations.

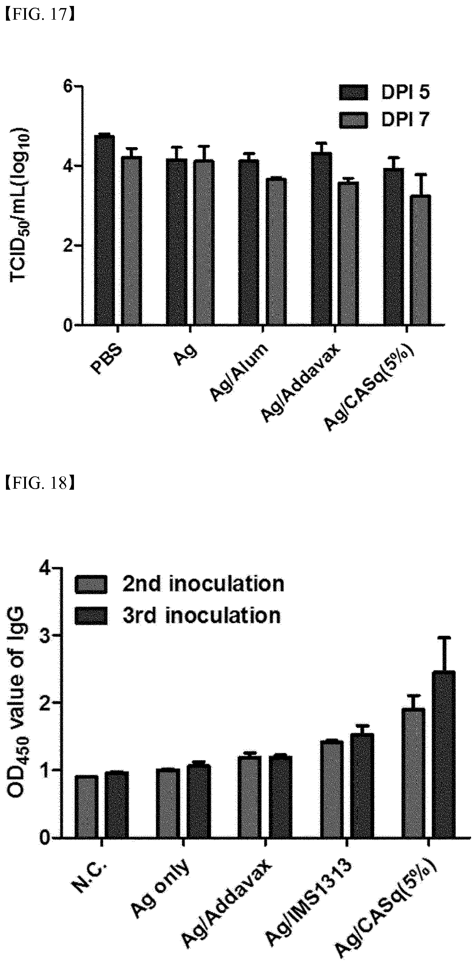

In addition, on days 5 and 7 of infection, the lungs of the mice were harvested to measure the titer of the virus remaining in the lungs. Here, three mice were investigated for each of the conditions. Results are shown in FIG. 17.

As shown in FIG. 17, it was confirmed that a weight change of the experimental group (Ag/CASq(5%)), in which the immunoadjuvant of the present invention was used, was the closest to that of the untreated control group, and the experimental group (Ag/CASq(5%)) exhibited a superior immunosuppressive ability compared to the commercially available immunoadjuvant. In addition, it was confirmed that, from results of the viral titers investigated on days 5 and 7 of infection, the lowest virus titer was exhibited upon use of the immunoadjuvant of the present invention.

### 3) Measurement of immunological activity in porcine epidemic diarrhea (PED) virus-infected guinea pig models

Guinea pigs were intramuscularly inoculated three times at 2-week intervals. At two weeks after the second and third inoculations, blood samples were collected from the guinea pigs. The collected blood samples were subjected to immunological activity measurement experiments using ELISA to investigate an antigen-specific antibody-based immune response (humoral immunity). To measure immunological activity in the guinea pigs, the collected serum was diluted 1:50, and the amount of IgG was investigated using enzyme-linked immunosorbent assay (ELISA). Experiments were conducted under the following five conditions: (i) an untreated group (N.C. of FIG. 18: administration of PBS 50 µl), ii) a PED immune vaccine antigen (Ag only of FIG. 18: administration of antigen 25 µl + PBS 25 µl), iii) a combination of PEDK (PED virus immune antigen for muscle inoculation) and a commercially available immunoadjuvant, AddaVax (InvivoGen, US) (Ag/AddaVax of FIG. 18: administration of antigen 25 µl + AddaVax 25 µl), iv) a combination of PEDK (PED virus immune antigen for muscle inoculation) and a commercially available immunoadjuvant, IMS1313 (SEPPIC, France) (Ag/IMS1313 of FIG. 18: administration of antigen 25 µl + IMS1313 25 µl), and v) a combination of PEDK (PED virus immune antigen for muscle inoculation) and squalene (5%)-carrying PLL-b-Phe (Ag/CASq(5%) of FIG. 18: administration of antigen 25 µl + CASq (5%) 25 µl). Here, the antigen and the immunoadjuvant were mixed in a ratio of 1:1 to prepare vaccine formulations. For each of the conditions, two mice were used as subjects. Results are shown in FIG. 18.

As shown in FIG. 18, it was confirmed that, when the immunoadjuvant according to the present invention was used, a remarkable IgG value was exhibited, compared to the case where a conventional immunoadjuvant used in a vaccine product against the porcine epidemic diarrhea (PED) virus was used. It was confirmed that, from the result of the immunological activity against the PED virus of the immunoadjuvant according to the present invention, the immunoadjuvant according to the present invention was an excellent immunoadjuvant against the PED virus.

## Claims

1. An amphiphilic poly-amino acid polymer represented by Formula 1 below: 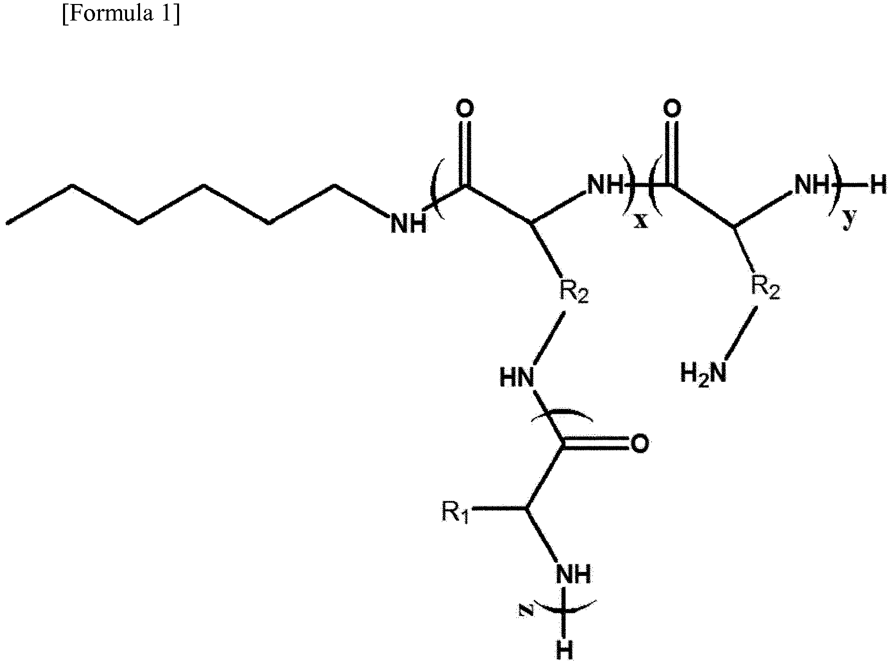 Wherein R₁ is one selected from the group consisting of 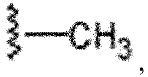 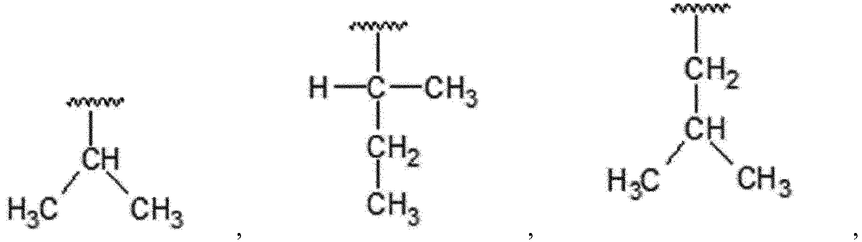 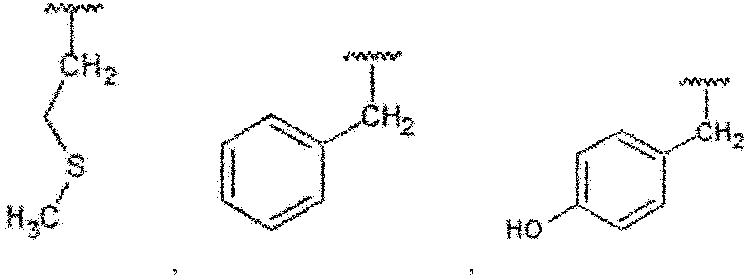 and 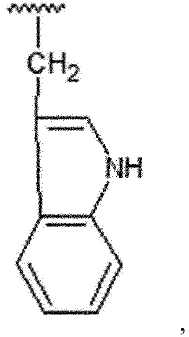 R₂ is 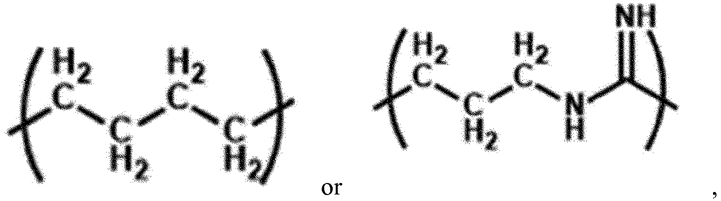 and
x, y, and z are integers greater than 1.

2. The amphiphilic poly-amino acid polymer according to claim 1, wherein the R₁ is 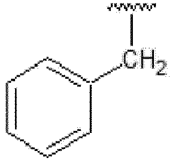 and the R₂ is

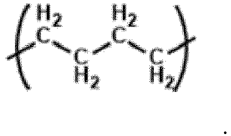

3. An immunoadjuvant composition, comprising squalene-carrying amphiphilic polymer particles in an oil-in-water emulsion state which contain the amphiphilic poly-amino acid polymer according to claim 1.

4. The immunoadjuvant composition according to claim 3, wherein the composition comprises squalene in an amount of 0.5% (v/v) to 10% (v/v).

5. The immunoadjuvant composition according to claim 3, wherein a molar ratio of the squalene to the amphiphilic poly-amino acid polymer is 1:0.001 to 1:0.999.

6. A vaccine composition, comprising an antigen; and squalene-carrying amphiphilic polymer particles in an oil-in-water emulsion state which contain the amphiphilic poly-amino acid polymer according to claim 1.

7. The vaccine composition according to claim 6, wherein the antigen is an influenza virus antigen or a porcine epidemic diarrhea virus antigen.

8. A method of preparing an amphiphilic poly-amino acid polymer, the method comprising a step of reacting a compound represented by Formula 2 below with a compound represented by Formula 3 below to prepare an amphiphilic poly-amino acid polymer represented by Formula 1: 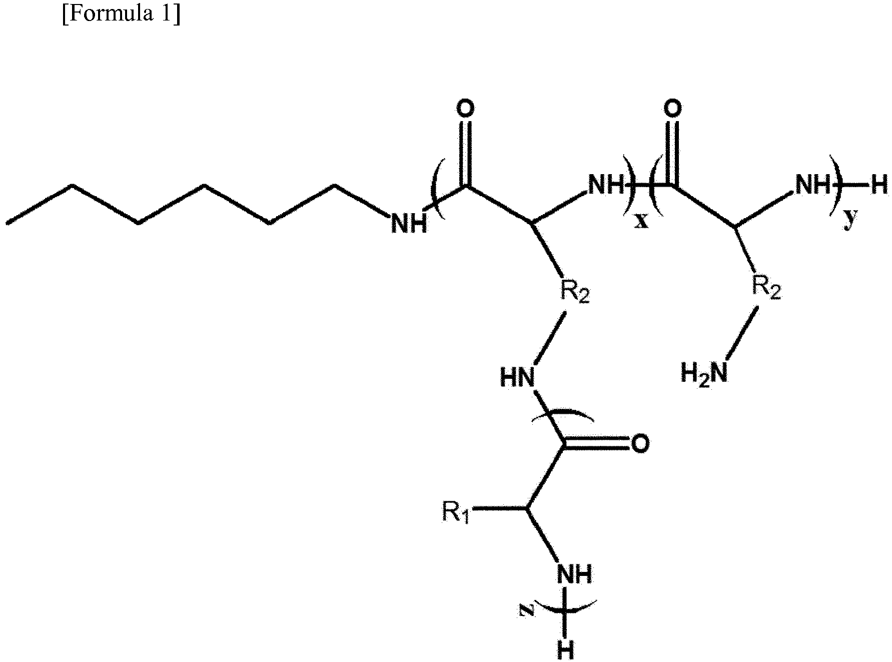 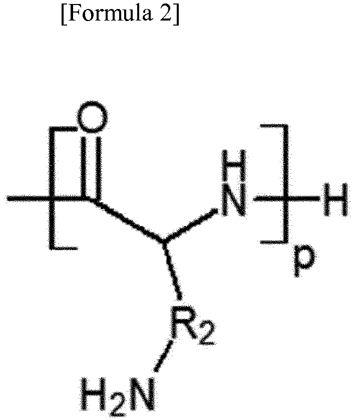 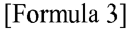 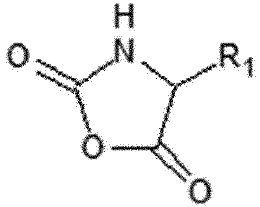 wherein, in Formulas 1 to 3,
R₁ is one selected from the group consisting of 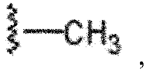 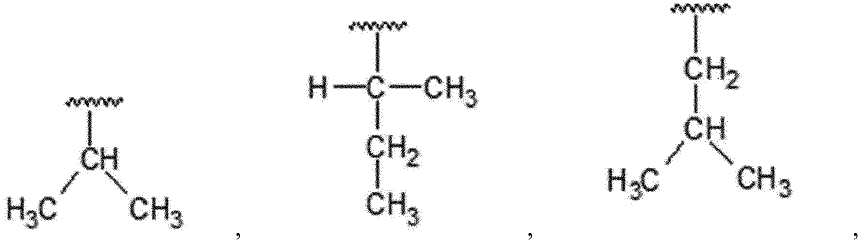 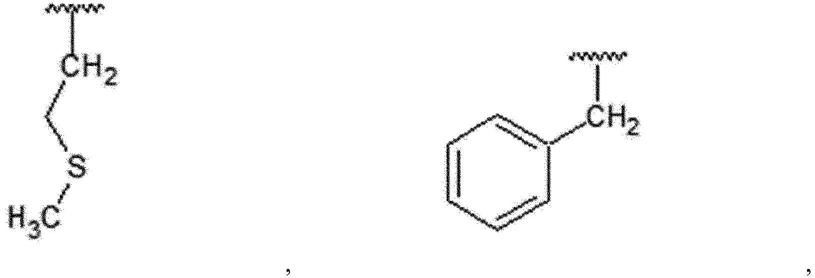 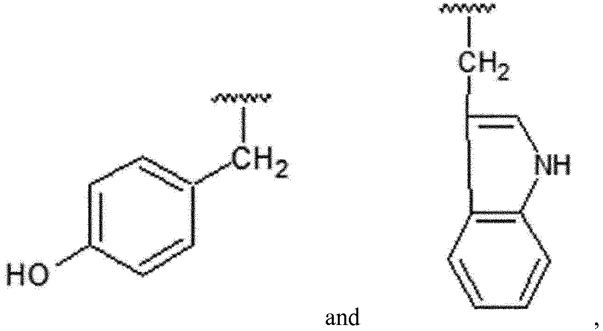 R₂ is 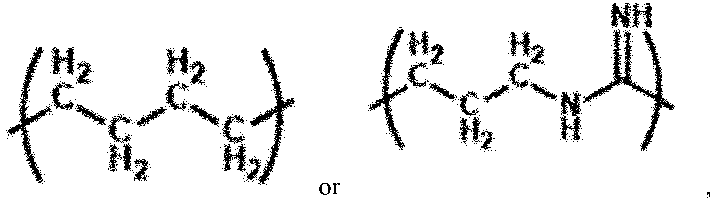 and
x, y, z, and p are integers greater than 1.

9. The method according to claim 8, wherein, in Formula 1, a ratio of x to y is 1:0.1 to 1:10.

10. A method of preparing an immunoadjuvant composition, the method comprising a step of mixing an aqueous solution comprising the amphiphilic poly-amino acid polymer according to claim 1 with a solution comprising squalene to prepare amphiphilic polymer particles in an oil-in-water emulsion state.

11. A method of preparing a vaccine composition, the method comprising a step of mixing a solution comprising an antigen with an emulsion solution comprising the squalene-carrying amphiphilic poly-amino acid polymer according to claim 1 which carries squalene.

12. The method according to claim 11, wherein, in the step, the solution comprising the antigen and the emulsion solution are mixed in a volumetric ratio of 1:0.2 to 1:5.

## Patentansprüche

1. Amphiphiles Polyaminosäurepolymer, dargestellt durch die nachstehende Formel 1: 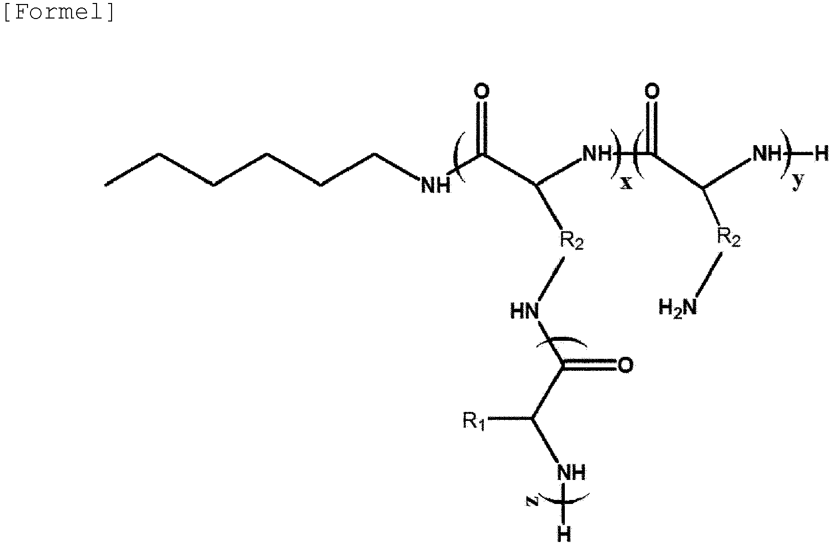 wobei R₁ eines ist, ausgewählt aus der Gruppe bestehend aus 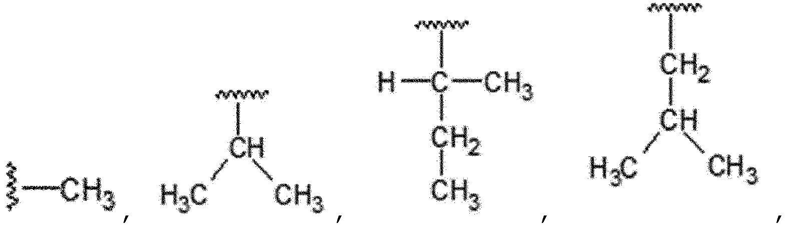 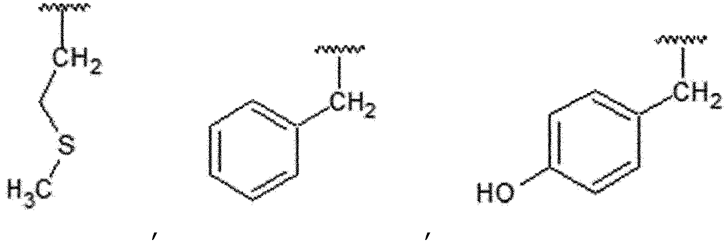 und 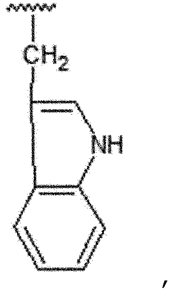 R₂ 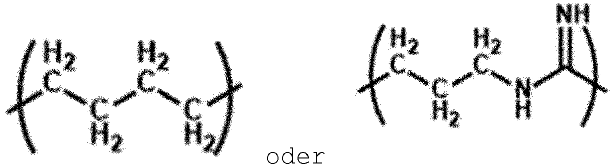 ist
und x, y und z ganze Zahlen größer 1 sind.

2. Amphiphiles Polyaminosäurepolymer nach Anspruch 1, wobei R₁ 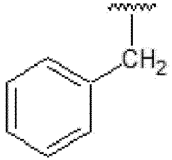 ist und R₂ 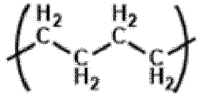 ist.

3. Immunoadjuvante Zusammensetzung, umfassend Squalen tragende amphiphile Polymerteilchen in einem Öl-in-Wasser-Emulsionszustand, die das amphiphile Polyaminosäurepolymer nach Anspruch 1 enhalten.

4. Immunoadjuvante Zusammensetzung nach Anspruch 3, wobei die Zusammensetzung Squalen in einer Menge von 0,5% (v/v) bis 10% (v/v) umfasst.

5. Immunoadjuvante Zusammensetzung nach Anspruch 3, wobei ein molares Verhältnis von dem Squalen zu dem amphiphilen Polyaminosäurepolymer 1:0,001 bis 1:0,999 beträgt.

6. Impfstoffzusammensetzung, umfassend ein Antigen; und Squalen tragende amphiphile Polymerteilchen in einem Öl-in-Wasser-Emulsionszustand, die das amphiphile Polyaminosäurepolymer nach Anspruch 1 enthalten.

7. Impfstoffzusammensetzung nach Anspruch 6, wobei das Antigen ein Influenzavirus-Antigen oder ein Antigen für das epidemische Durchfallvirus bei Schweinen ist.

8. Verfahren zur Herstellung eines amphiphilen Polyaminosäurepolymers, wobei das Verfahren einen Schritt zum Umsetzen einer durch die nachstehende Formel 2 dargestellten Verbindung mit einer durch die nachstehende Formel 3 dargestellten Verbindung umfasst, um ein durch die Formel 1 dargestelltes amphiphiles Polyaminosäurepolymer herzustellen: 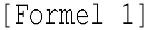 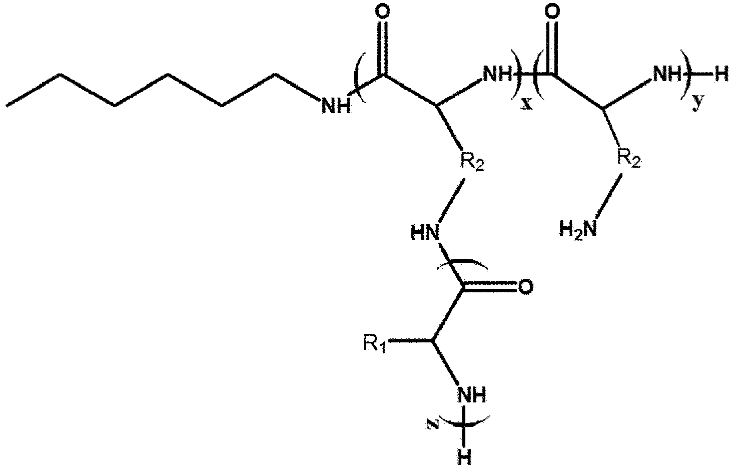 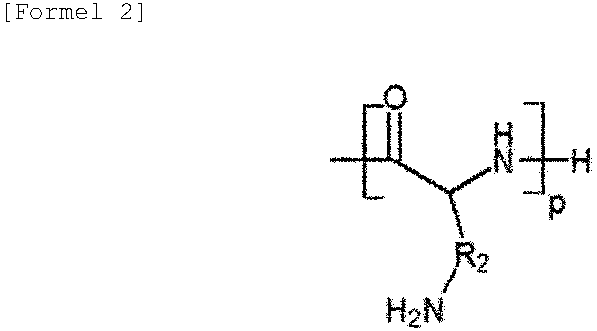 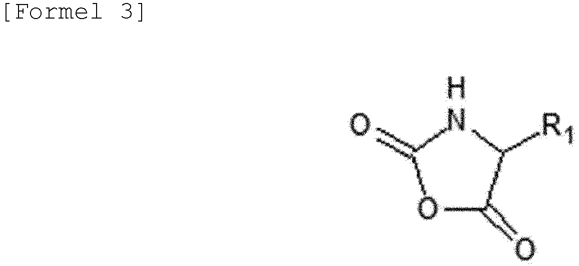 wobei in den Formeln 1 bis 3
R₁ eines ist, ausgewählt aus der Gruppe bestehend aus 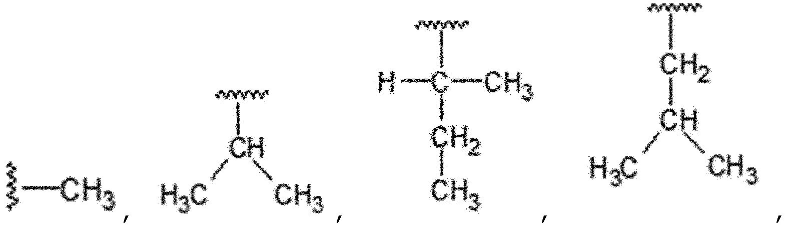 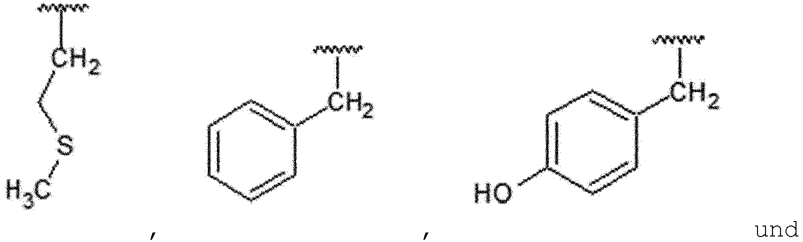 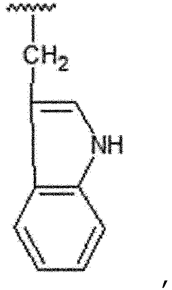 R₂ 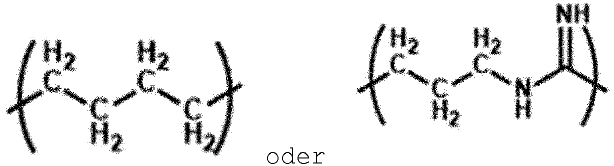 ist
und x, y, z und p ganze Zahlen größer 1 sind.

9. Verfahren nach Anspruch 8, wobei in Formel 1 ein Verhältnis von x zu y 1:0,1 bis 1:10 beträgt.

10. Verfahren zur Herstellung einer immunoadjuvanten Zusammensetzung, wobei das Verfahren einen Schritt des Mischens einer wässrigen Lösung, umfassend das amphiphile Polyaminosäurepolymer nach Anspruch 1, mit einer Lösung, umfassend Squalen, umfasst, um amphiphile Polymerteilchen in einem Öl-in-Wasser-Emulsionszustand herzustellen.

11. Verfahren zur Herstellung einer Impfstoffzusammensetzung, wobei das Verfahren einen Schritt des Mischens einer Lösung, umfassend ein Antigen, mit einer Emulsionslösung, umfassend das Squalen tragende amphiphile Polyaminosäurepolymer nach Anspruch 1, welches Squalen trägt, umfasst.

12. Verfahren nach Anspruch 11, wobei in dem Schritt die Lösung, die das Antigen umfasst, und die Emulsionslösung in einem Volumenverhältnis von 1:0,2 bis 1:5 gemischt werden.

## Revendications

1. Polymère amphiphile d'acide aminé représenté par la formule 1 ci-dessous : 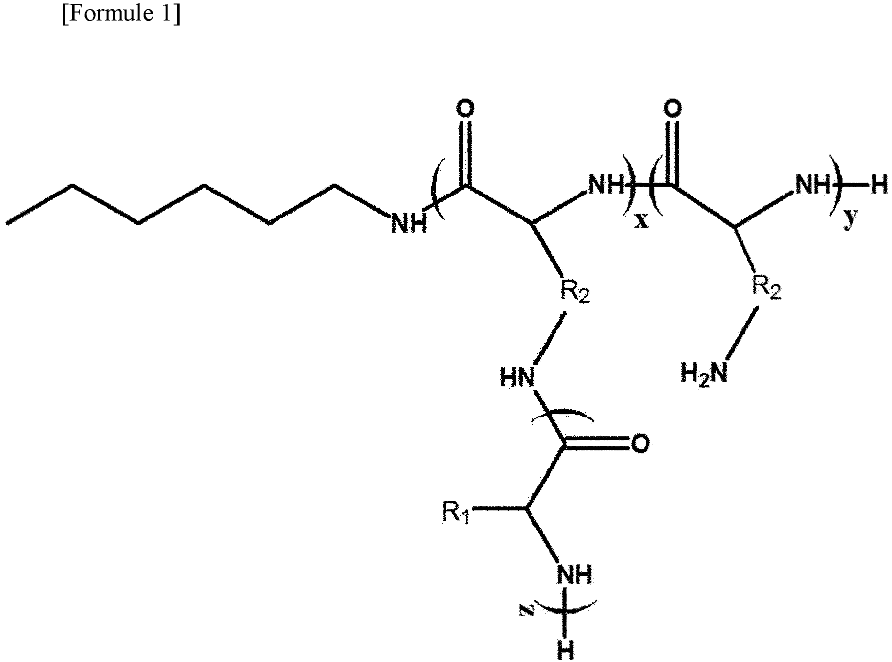 Où R₁ est choisi dans le groupe constitué par 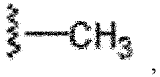 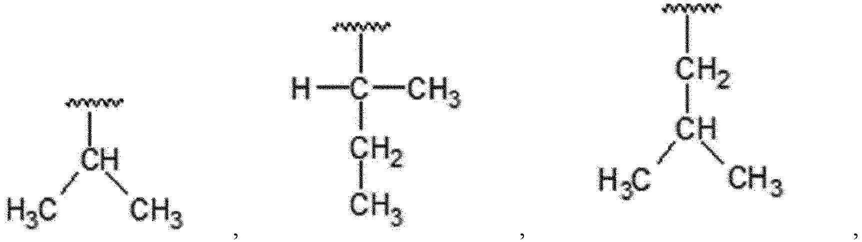 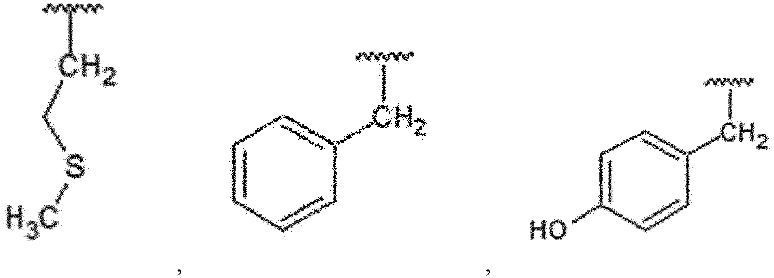 et 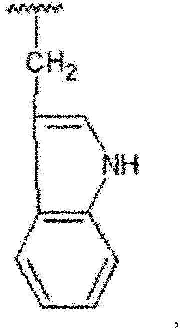 R₂ est 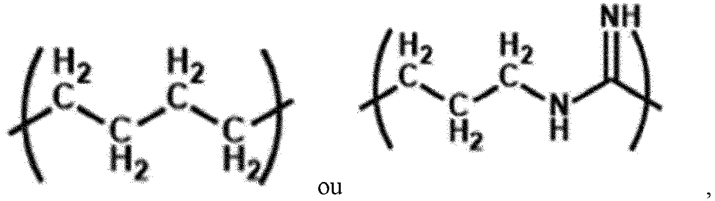 et
x, y et z sont des entiers supérieurs à 1.

2. Polymère amphiphile de polyacide aminé selon la revendication 1, dans lequel R₁ est 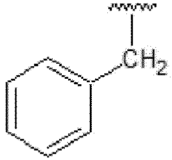 et R₂ est

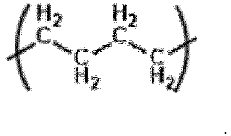

3. Composition immunoadjuvante, comprenant des particules de polymère amphiphile portant du squalène dans un état d'émulsion huile-dans-eau qui contiennent le polymère de poly(acide aminé) amphiphile selon la revendication 1.

4. Composition immunoadjuvante selon la revendication 3, dans laquelle la composition comprend du squalène en une quantité de 0,5% (v/v) à 10% (v/v).

5. Composition immunoadjuvante selon la revendication 3, dans laquelle un rapport molaire du squalène au polymère de polyacide aminé amphiphile est de 1:0,001 à 1:0,999.

6. Composition de vaccin, comprenant un antigène ; et des particules de polymère amphiphile portant du squalène dans un état d'émulsion huile-dans-eau qui contiennent le polymère de poly(acide aminé) amphiphile selon la revendication 1.

7. Composition de vaccin selon la revendication 6, dans laquelle l'antigène est un antigène de virus de la grippe ou un antigène de virus de la diarrhée épidémique porcine.

8. Procédé de préparation d'un polymère de poly-acide aminé amphiphile, le procédé comprenant une étape de réaction d'un composé représenté par la formule 2 ci-dessous avec un composé représenté par la formule 3 ci-dessous pour préparer un polymère de poly-acide aminé amphiphile représenté par la formule 1 : 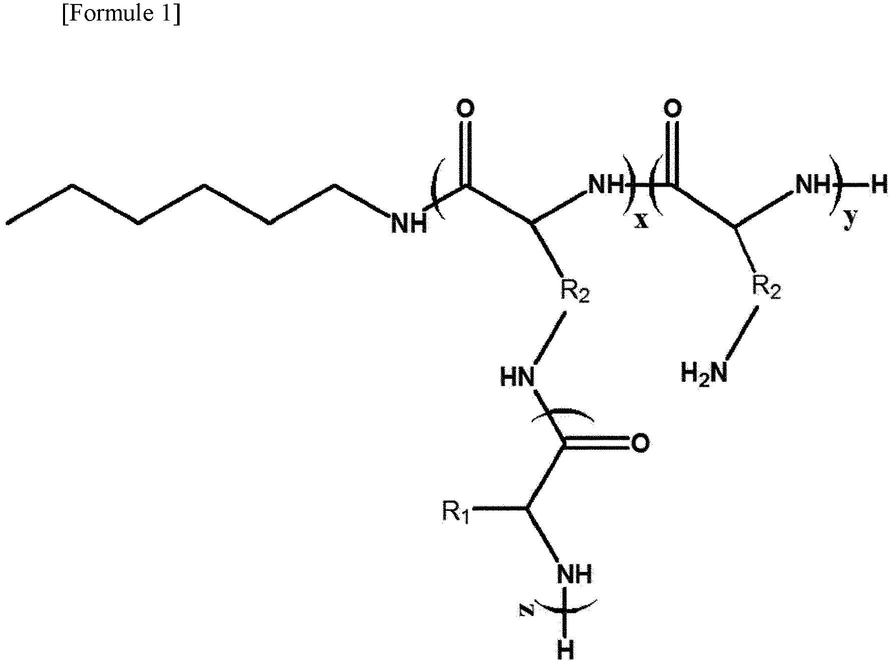 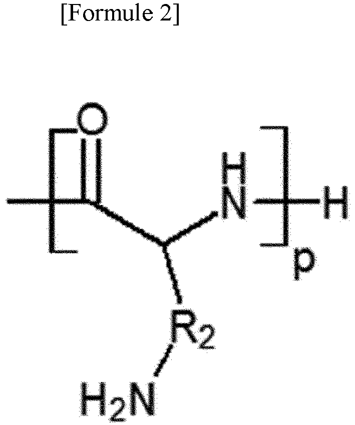 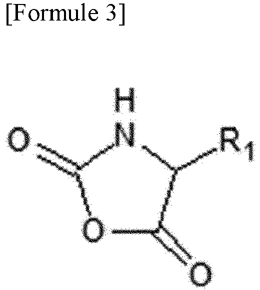 où, dans les formules 1 à 3,
R₁ est choisi dans le groupe constitué par 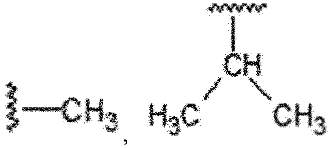 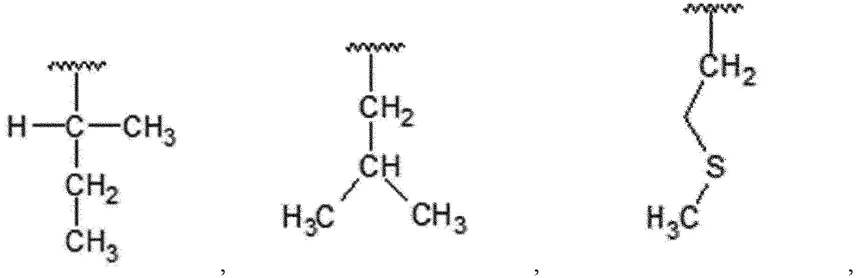 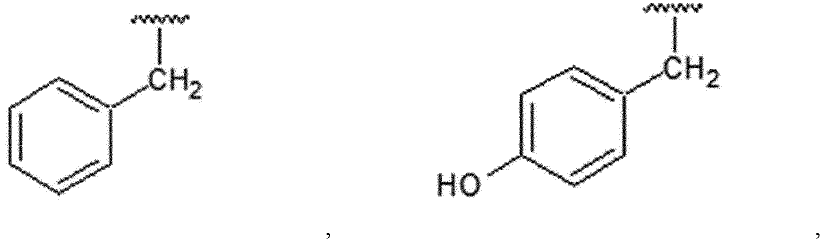 et 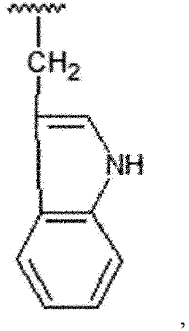 R₂ est 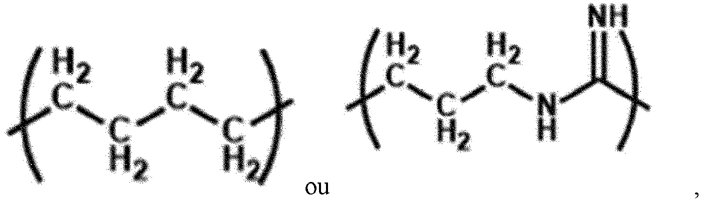 et
x, y, z et p sont des entiers supérieurs à 1.

9. Procédé selon la revendication 8, dans laquelle, dans la formule 1, un rapport de x à y est de 1:0,1 à 1:10.

10. Procédé de préparation d'une composition immunoadjuvante, le procédé comprenant une étape de mélange d'une solution aqueuse comprenant le polymère de poly(acide aminé) amphiphile selon la revendication 1 avec une solution comprenant du squalène pour préparer des particules de polymère amphiphile dans un état d'émulsion huile dans l'eau.

11. Procédé de préparation d'une composition de vaccin, le procédé comprenant une étape de mélange d'une solution comprenant un antigène avec une solution d'émulsion comprenant le polymère de poly-acide aminé amphiphile porteur de squalène selon la revendication 1 qui porte le squalène.

12. Procédé selon la revendication 11, dans laquelle, dans l'étape, la solution comprenant l'antigène et la solution d'émulsion sont mélangées dans un rapport volumétrique de 1:0,2 à 1:5.
